# EUROPÄISCHE PATENTSCHRIFT

(11) **EP 2 349 408 B1**
(45) Veröffentlichungstag und Bekanntmachung des Hinweises auf die Patenterteilung: **11.11.2015**
(21) Anmeldenummer: 09775789.2
(22) Anmeldetag: 18.09.2009
(51) Int. Cl.: A61M 5/178

(54) **INJEKTIONSVORRICHTUNG MIT EXAKT FESTSTELLBARER KOLBENSTANGE**
INJECTION DEVICE WITH AN EXACTLY ADJUSTABLE PLUNGER ROD
DISPOSITIF INJECTEUR DOTÉ D'UNE TIGE DE PISTON DE PRÉCISION

(30) Priorität: 30.09.2008 DE 102008049638
(43) Veröffentlichungstag der Anmeldung: 03.08.2011
(73) Patentinhaber: Tecpharma Licensing AG, 3401 Burgdorf (CH)
(72) Erfinder: MOSER, Ueli, 3412 Heimiswil (CH); HIRSCHEL, Jürg, 3007 Bern (CH); TSCHIRREN, Markus, 3400 Burgdorf (CH)
(86) Internationale Anmeldenummer: PCT/CH2009/000306
(87) Internationale Veröffentlichungsnummer: WO 2010/037241

(56) Entgegenhaltungen:
- WO-A1-97/09080
- WO-A1-2005/072797
- US-A- 5 281 198

## Beschreibung

Die Erfindung bezieht sich auf eine Injektionsvorrichtung, welche bevorzugt zur Selbstverabreichung einer Substanz oder eines Medikaments verwendet werden kann Insbesondere bezieht sich die Erfindung auf eine Injektionsvorrichtung, welche in Verbindung mit einer Mehrkammer- oder Zweikammer-Ampulle verwendet werden kann.

Mittels Mehrkammer-Ampullen, insbesondere Zweikammer-Ampullen, können Produkte und insbesondere fluide Produkte verabreicht werden, welche aus mehreren Komponenten bestehen, die erst kurz vor der Verabreichung abgemischt werden. Dazu ist zum Beispiel in einer ersten Kammer eine Flüssigkeit als Lösungsmittel und in einer zweiten Kammer eine Produktkomponente in festem oder auch flüssigem Zustand vorgesehen. Das Lösungsmittel und die Produktkomponente werden in der Ampulle mittels einer Abmischvorrichtung, zum Beispiel durch Einbringen der Ampulle in die Injektionsvorrichtung, abgemischt. Mit Hilfe der Abmischvorrichtung kann das zu injizierende fluide Produkt kurz vor einer Verabreichung abgemischt werden, indem ein Stopfen der Zweikammer-Ampulle innerhalb der Ampulle derart verschoben wird, dass das Lösungsmittel über einen Zufuhrkanal mit der Produktkomponente in Kontakt kommt und sich mit dieser vermischt.

Zweikammer-Ampullen sind zum Beispiel aus der WO 2006/012770 A1 bekannt.

Wird eine zum Beispiel in einen Ampullenhalter eingesetzte Zweikammer-Ampulle zum Abmischen in eine Injektionsvorrichtung eingeschraubt, so gelangt der proximale Stopfen der Zweikammer-Ampulle in Kontakt mit einem vorderen oder distalen Ende einer Kolbenstange, welche auf den proximalen Stopfen drückt und diesen beim weiteren Einschieben oder Eindrehen des Ampullenhalters in die Injektionsvorrichtung relativ zu dem Ampullenkörper in die Ampulle einschiebt, um den Abmischvorgang zu bewirken.

Wird eine Injektionsvorrichtung verwendet, bei welcher die Kolbenstange zusammen mit dem Injektionsknopf durch den Ampullenhalter in proximale Richtung verschoben wird, so kann der Start der Bewegung der Kolbenstange kraftabhängig gesteuert werden. Eine kraftabhängige Steuerung ist bezüglich äußerer Einflüsse jedoch störanfällig. Die Position der Kolbenstange innerhalb der Injektionsvorrichtung ist wesentlich für die Genauigkeit der Ausschüttung, so dass eine hohe Ausschüttgenauigkeit mit einer ungenau positionierten und zum Beispiel durch eine Kraftsteuerung verschobenen Kolbenstange nicht gewährleistet werden kann.

Ein Einsetzelement gemäß Oberbegriff von Patentanspruch 1 ist z.B. aus WO 97/09080 bekannt.

Es ist eine Aufgabe der vorliegenden Erfindung eine mit einem Injektionsknopf zusammenwirkende Kolbenstange sowie eine Injektionsvorrichtung vorzuschlagen, welche eine hohe Ausschüttgenauigkeit, insbesondere bei einer vorher abgemischten Mehrkammer-Ampulle, ermöglichen.

Diese Aufgabe wird durch die Gegenstände der unabhängigen Ansprüche gelöst. Vorteilhafte Ausführungsformen sind in den abhängigen Ansprüchen definiert.

Erfindungsgemäß wird eine Kolbenstange für eine Injektionsvorrichtung vorgesehen, welche so ausgebildet ist, dass sie von einem Positionierungselement und insbesondere einem mit dem Gehäuse der Injektionsvorrichtung verbundenen Element auch beim Einbringen oder Einschrauben eines Ampullenhalters oder einer Mehrkammer-Ampulle in axialer Position relativ zu dem Gehäuse der Injektionsvorrichtung zum Beispiel mittels eines an der Kolbenstange vorgesehenen Abstützelements gehalten werden kann, um eine exakte Position der Kolbenstange auch nach erfolgtem Einbringen der Mehrkammer-Ampulle definieren und sicherstellen zu können. Durch die exakte Positionierung und bevorzugt axiale mindestens einseitige Feststellung der Kolbenstange kann die Position der Kolbenstange nach dem Abmischvorgang der Mehrkammer-Ampulle und somit auch die Position des an der Kolbenstange anliegenden proximalen Stopfens vor der Injektion exakt definiert werden, wodurch eine hohe Ausschüttgenauigkeit erreicht werden kann. Somit kann die Kolbenstange zum Beispiel durch Anlage an einem innerhalb des Gehäuses befindlichen Halteelement weggesteuert in axialer Richtung positioniert werden. Die Kolbenstange bleibt während dem Abmischvorgang relativ zu dem Gehäuse immer in derselben axialen Position.

Vorzugsweise ist ein Injektionsknopf mit der Kolbenstange verbunden, wobei die Verbindung des Injektionsknopfs mit der Kolbenstange unmittelbar oder über ein Federelement sein kann. Vorteilhaft werden Kolbenstange und Injektionsknopf und optional auch das zwischen Kolbenstange und Injektionsknopf angeordnete Federelement aus dem gleichen Material oder einteilig gefertigt. Ist zwischen der Kolbenstange und dem Injektionsknopf ein Federelement vorgesehen, also der Injektionsknopf mit der Kolbenstange zum Beispiel durch das dazwischen angeordnete Federelement verbunden, ist es möglich die Bewegungen der Kolbenstange und des Injektionsknopfs unabhängig voneinander zu steuern, so dass zum Beispiel die Kolbenstange während eines Abmischvorgangs in einer festgelegten gleichen Position relativ zum Gehäuse bleibt, wohingegen sich der Injektionsknopf, zum Beispiel geschoben oder bewegt durch die Bewegung des Ampullenhalters, aus dem Gehäuse bewegen kann, wenn zum Beispiel der Injektionsknopf vor dem Abmischvorgang vollständig oder fast vollständig in dem Gehäuse der Injektionsvorrichtung gelagert ist.

Werden die Kolbenstange und der Injektionsknopf und optional auch das zusätzlich vorgesehene Federelement einteilig und zum Beispiel aus dem gleichen Material zum Beispiel in einem Spritzgussverfahren gefertigt, so kann eine Sollbruchstelle an einer Verbindung zwischen dem Injektionsknopf und der Kolbenstange vorgesehen werden, welche zum Beispiel den Injektionsknopf in einer solchen Position an der Kolbenstange hält, das zum Beispiel das zwischen der Kolbenstange und dem Injektionsknopf vorgesehene Federelement komprimiert ist. Wird die Sollbruchstelle zum Beispiel während oder nach dem Einsetzen der Kolbenstange und des damit verbundenen Injektionsknopfs in die Injektionsvorrichtung oder in das Gehäuse der Injektionsvorrichtung geöffnet, kann der nicht mehr über die geöffnete Sollbruchstelle mit der Kolbenstange verbundene Injektionsknopf durch das Federelement an der Kolbenstange gehalten und somit vor einem Herausfallen aus der Injektionsvorrichtung gesichert werden, wobei jedoch die Kolbenstange und der Injektionsknopf in einem durch zum Beispiel durch die Federlänge definierten Bereich weitgehend unabhängig voneinander bewegt werden können.

Die Erfindung bezieht sich gemäß einem weiteren Aspekt auf eine Injektionsvorrichtung mit einem Gehäuse und einer wie oben beschriebenen Anordnung bestehend aus Kolbenstange, Injektionsknopf und optional vorgesehenem Federelement. Dabei ist die Injektionsvorrichtung vorteilhaft so ausgebildet, dass die beschriebene Anordnung in die Injektionsvorrichtung zum Beispiel an proximaler Seite der Injektionsvorrichtung in die Injektionsvorrichtung eingebracht und zum Beispiel eingeschoben werden kann. Dabei kann das Gehäuse oder die Injektionsvorrichtung so ausgebildet sein, dass während oder nach dem Einbringen oder Einschieben der Anordnung eine Sollbruchstelle zwischen der Kolbenstange und dem Injektionsknopf geöffnet wird.

Die Erfindung wird nachfolgend anhand eines Ausführungsbeispiels beschrieben. Es zeigen:
- Figur 1A: ein Gehäuse einer Injektionsvorrichtung mit Ampullenhalter im Auslieferungszustand;
- Figur 1B: einen Querschnitt der in Figur 1A gezeigten Injektionsvorrichtung in Zeichenebene;
- Figur 1C: einen Querschnitt der in Figur 1A gezeigten Injektionsvorrichtung in einer Richtung orthogonal zur der in Figur 1B gezeigten Darstellung;
- Figur 2A: die in Figur 1A gezeigte Injektionsvorrichtung in abgemischtem Zustand;
- Figur 2B: einen Querschnitt der in Figur 2A gezeigten Injektionsvorrichtung in Zeichenebene;
- Figur 2C: einen Querschnitt der in Figur 2A gezeigten Injektionsvorrichtung in einer Richtung orthogonal zur der in Figur 2B gezeigten Darstellung;
- Figur 2D: eine Detaildarstellung der proximalen Seite der Injektionsvorrichtung;
- Figur 3A: die in Figur 2A gezeigte Injektionsvorrichtung gegen Ende der Ausschüttung;
- Figur 3B: einen Querschnitt der in Figur 3A gezeigten Injektionsvorrichtung in Zeichenebene; und
- Figur 3C: ein Querschnitt der in Figur 3A gezeigten Injektionsvorrichtung in einer Richtung orthogonal zu der in Figur 3B gezeigten Darstellung.

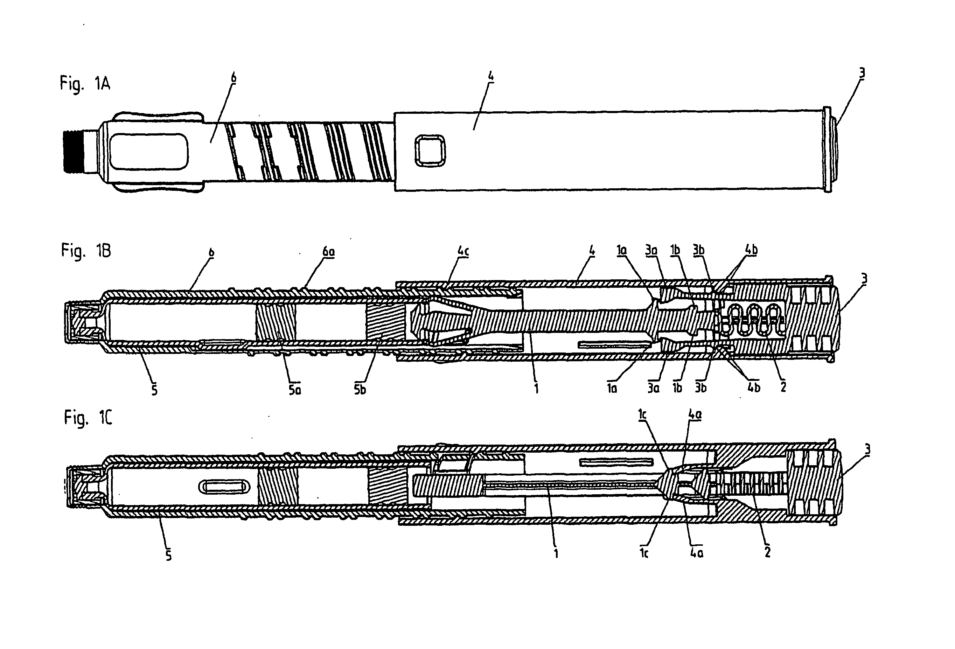

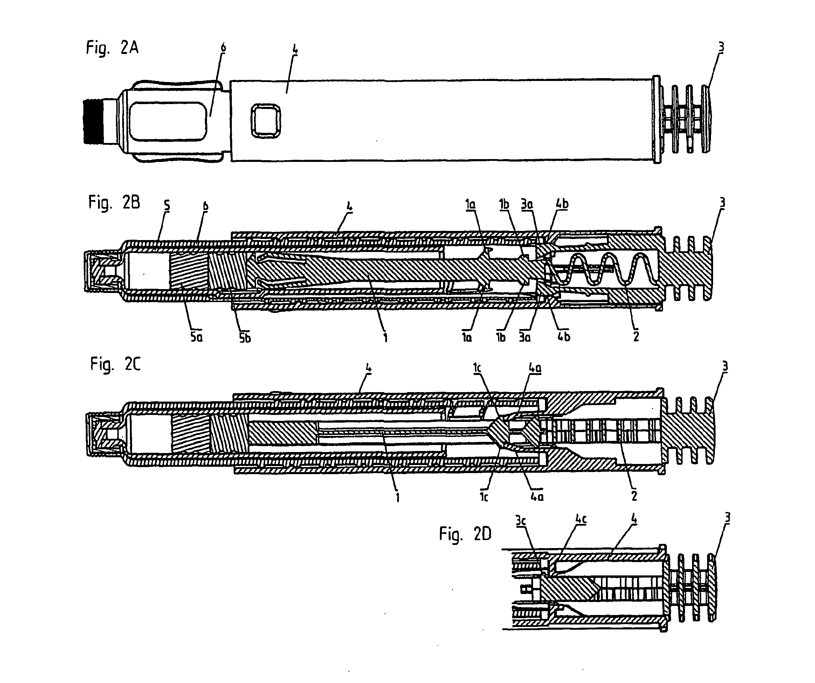

Figur 1A zeigt ein Gehäuse 4 einer Injektionsvorrichtung, in welche ein Ampullenhalter 6 mit darin eingesetzter Zweikammer-Ampulle 5 zum Abmischen der Zweikammer-Ampulle 5, wie in Figur 1B und 2B gezeigt, eingeschraubt werden kann.

Der Ampullenhalter 6 weist auf seiner Außenseite ein Gewinde 6a auf, welches in ein korrespondierendes Innengewinde 4c des Gehäuses 4 eingreifen kann. In dem in Figur 1 gezeigten Auslieferungszustand ist das im Ausführungsbeispiel einteilige Einsetzelement bestehend aus Kolbenstange 1, Feder 2 und Injektionsknopf 3 von der in Figur 1 rechts gezeigten proximalen Seite in die Injektionsvorrichtung eingesetzt worden.

Wie aus Figur 1C ersichtlich, sind an der Kolbenstange 1 in radiale Richtung vorstehend Anschlagelemente 1c vorgesehen, welche sich gegen auf der Gehäuseinnenseite befindliche fest mit dem Gehäuse 4 verbundene Arme 4a abstützen und so die Kolbenstange 1 gegen ein Verschieben in proximale Richtung sichern. Vorteilhaft sind die Halteelemente 4a als Federarme so ausgebildet, dass die Kolbenstange 1 einschließlich der Halteelemente 1 c von proximaler Seite her in distale Richtung in das Gehäuse 4 eingeschoben werden kann, wobei die Federarme 4a radial nach außen verdrängt werden, so dass die Haltelemente 1c an den Gehäusearmen 4a vorbeigeschoben werden können. Ist die Kolbenstange 1 soweit in die Injektionsvorrichtung eingeschoben worden, dass die Halteelemente 1 c an den elastischen oder Federarmen 4a des Gehäuses 4 vorbeigeschoben wurden, so schnappen die Gehäusearme 4a zum Beispiel auf Grund ihrer radial nach innen gerichteten Vorspannung wieder in die Ausgangsposition zurück, wie beispielsweise in Figur 1C gezeigt, so dass die Kolbenstange 1 durch die Gehäusearme 4a gegen eine axiale Verschiebung in proximale Richtung gesichert ist.

In dem in Figur 1 gezeigten Auslieferungszustand ist der Injektionsknopf 3 mittels des Federelements 2 mit der Kolbenstange 1 verbunden, wobei eine zwischen einem radialen Vorsprung 1 a der Kolbenstange 1 und einem Element oder Schnapparm 3a des Injektionsknopfs 3 vorgesehene Sollbruchstelle noch nicht geöffnet oder auch bereits geöffnet sein kann. Der Injektionsknopf 3 weist Schnapparme 3a auf, welche Vorsprünge 3b haben, die mit einem Verdrängungselement, zum Beispiel einer schrägen Fläche 4b des Gehäuses 4 so zusammenwirken können, dass beispielsweise bei einem Druck auf den Injektionsknopf 3 in proximale Richtung zum Beispiel durch den Druck des eingeschraubten Ampullenhalters 6 auf die Schnapparme 3a, die Schnapparme 3a zum Beispiel radial nach innen ausgelenkt werden; wodurch beispielsweise die beschriebene Sollbruchstelle geöffnet werden kann, so dass der Injektionsknopf 3 unabhängig von der Kolbenstange 1 bewegt und zum Beispiel aus dem Gehäuse 4 der Injektionsvorrichtung herausgeschoben werden kann, wie in Figur 2 gezeigt, wobei gleichzeitig jedoch die Kolbenstange 1, gehalten durch die Gehäusearme 4a, in einer definierten axialen Position bleibt.

Beim Einschrauben des Ampullenhalters 6 in das Gehäuse 4 drückt die in ihrer axialen Position gehaltene Kolbenstange 1 mit ihrem distalen Ende auf den proximalen Stopfen 5b der Ampulle 5 und verschiebt diesen relativ zu Ampulle 5 während des weiteren Eindrehens des Ampullenhalters 6 in das Gehäuse 4 in distale Richtung auf den zweiten Stopfen 5a, bis der ersten Stopfen 5b an dem zweiten Stopfen 5a anliegt, wie in Figur 2B gezeigt, wodurch die Zweikammer-Ampulle 5 abgemischt wird.

Dabei kommt der Ampullenhalter 6 mit den Armen 3a des Injektionsknopfs 3 in Kontakt und schiebt diesen aus der Injektionsvorrichtung heraus. Der Injektionsknopf 3 wird durch das Federelement 2 an der axial in proximale Richtung nicht verschiebbaren Kolbenstange 1 gehalten, sowie durch die Vorsprünge 3b vor dem Herausfallen gesichert.

Wie in Figur 2B gezeigt, liegen im abgemischten Zustand die Arme 3a des Injektionsknopfs 3 an bezüglich der oben erwähnten Vorsprünge 1a in proximale Richtung beabstandeten zweiten Vorsprüngen 1b der Kolbenstange 1 an. Wird der aus der Injektionsvorrichtung ausgeschobene Injektionsknopf 3 eingedrückt, so kann die Einschubbewegung des Injektionsknopfs 3 über die vorteilhaft radial nach innen vorgespannten Arme 3a, welche an den Vorsprüngen 1 b der Kolbenstange 1 anliegen, auf die Kolbenstange 1 übertragen werden, so dass die Kolbenstange 1 durch Druck auf den Injektionsknopf 3 in distale Richtung bewegt wird und somit die Stopfen 5a und 5b in distale Richtung in die Ampulle 5 einschiebt, wodurch die abgemischte Substanz aus der Ampulle 5 verdrängt und somit injiziert werden kann.

Da die Kolbenstange 1 während des Abmischvorgangs in einer exakt definierten axialen Position gehalten wird, kann eine hohe Ausschüttgenauigkeit erreicht werden.

Wie aus Figur 2D ersichtlich, weisen die Schnapparme 3a seitlich je einen Nocken 3c auf, welche gegen ein weiteres Herausziehen des Injektionsknopfs 3 am Gehäuseboden 4c anstehen.

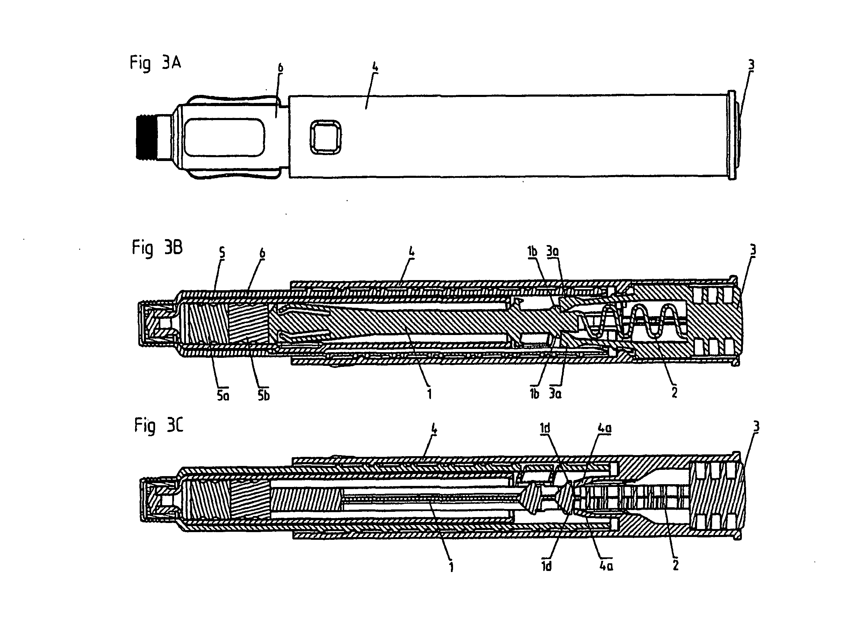

In Figur 3 ist die in Figur 2 gezeigte Injektionsvorrichtung gegen Ende der Ausschüttung gezeigt. Wie in Figur 3C ersichtlich, sind an der Kolbenstange 1 in radiale Richtung vorstehende Rampen 1 d vorgesehen, über welche die Halteelemente 4a gegen Ende der Ausschüttung ausgelenkt werden und anschließend einschnappen, wodurch ein akustisches Geräusch verursacht wird, welches einem Benutzer signalisieren kann, dass das Ende der Injektion erreicht ist.

## Patentansprüche

1. Einsetzelement für eine Injektionsvorrichtung mit einer Kolbenstange (1) und einem mit der Kolbenstange (1) verbundenen und/oder zusammenwirkenden Injektionsknopf (3), wobei die Kolbenstange (1) ein Abstützelement (1c) aufweist, mit welchem die Kolbenstange (1) gegen ein axiales Verschieben in proximale Richtung der Injektionsvorrichtung gehalten werden kann, wobei die Kolbenstange (1) über ein elastisches oder Federelement (2) mit dem Injektionsknopf (3) verbunden ist, **dadurch gekennzeichnet, dass** die Kolbenstange (1), das elastische- oder Federelement (2) und der Injektionsknopf (3) einteilig sind.

2. Einsetzelement nach einem der vorhergehenden Ansprüche, wobei eine Sollbruchstelle (1a, 3a) zwischen der Kolbenstange (1) und dem injektionsknopf (3) vorgesehen ist.

3. Einsetzelement nach einem der vorhergehenden Ansprüche, wobei der Injektionsknopf (3) relativ zur Kolbenstange (1) verschiebbar oder bewegbar ist

4. Einsetzelement nach einem der vorhergehenden Ansprüche, wobei die Kolbenstange (1) mindestens zwei Kraftaufnahmeelemente (1a, 1b) aufweist.

5. Einsetzelement nach einem der vorhergehenden Ansprüche, wobei der Injektionsknopf (3) ein Kraftübertragungselement (3a) aufweist, mit welchem eine auf den Injektionsknopf (3) wirkende Kraft auf die Kolbenstange (1) oder ein Kolbenstangenelement (1a) übertragen werden kann.

6. Injektionsvorrichtung mit einem Gehäuse (4) und einem Einsetzelement (1, 2, 3) nach einem der vorgehenden Ansprüche.

7. Injektionsvorrichtung nach dem vorgehenden Anspruch mit mindestens einem Halteelement (4a), mit welchem die Kolbenstange (1) in einer axialen Position gegen eine in proximale Richtung wirkende Kraft gehalten werden kann.

8. Injektionsvorrichtung nach einem der zwei vorhergehenden Ansprüche mit mindestens einem Verdrängungselement (4b), welches ein Element oder einen Arm (3a) des Injektionsknopfs in radiale Richtung der Injektionsvorrichtung (3) verdrängen oder verschieben kann, wenn auf den Arm (3a) eine axiale Kraft wirkt.

9. Injektionsvorrichtung nach einem der drei vorhergehenden Ansprüche, welche so ausgebildet ist, dass das Einsetzelement (1, 2, 3) nach einem der Ansprüche 1 bis 5 von proximaler Seite in das Gehäuse (4) der Injektionsvorrichtung eingesetzt werden kann.

## Claims

1. Insertion element for an injection device with a piston rod (1) and an injection button (3) connected and/or cooperating with the piston rod (1), whereby the piston rod (1) comprises a support element (1c), with which the piston rod (1) can be held against an axial displacement in a proximal direction of the injection device, whereby the piston rod (1) is connected with the injection button (3) via an elastic or spring element (2) **characterised in that** the piston rod (1), the elastic or spring element (2) and the injection button (3) are one piece.

2. Insertion element according to one of the preceding claims, whereby a predetermined breaking point (1a, 3a) is envisaged between the piston rod (1) and the injection button (3).

3. Insertion element according to one of the preceding claims, whereby the injection button (3) can be displaced or moved relative to the piston rod (1).

4. Insertion element according to one of the preceding claims, whereby the piston rod (1) comprises at least two force absorption elements (1a, 1b).

5. Insertion element according to one of the preceding claims, whereby the injection button (3) comprises a force transmission element (3a), with which a force acting on the injection button (3) can be transferred to the piston rod (1) or a piston rod element (1a).

6. Injection device with a housing (4) and an insertion element (1, 2, 3) according to one of the preceding claims.

7. Injection device according to the preceding claim, with at least one holding element (4a), with which the piston rod (1) can be held in an axial position against a force acting in a proximal direction.

8. Injection device according to one of the two preceding claims, with at least one displacement element (4b), which can displace or push an element or an arm (3a) of the injection button in a radial direction of the injection device (3) when an axial force acts on the arm (3a).

9. Injection device according to one of the three preceding claims, designed in such a way that the insertion element (1, 2, 3) can be inserted into the housing (4) of the injection device from the proximal side according to one of the claims 1 to 5.

## Revendications

1. Élément d'insertion pour un dispositif d'injection comportant une tige de piston (1) et un bouton d'injection (3) relié et/ou coopérant avec la tige de piston (1), ladite tige de piston (1) comportant un élément d'appui (1c), par lequel la tige de piston (1) peut être empêchée d'effectuer un déplacement axial dans la direction proximale du dispositif d'injection, ladite tige de piston (1) étant reliée au bouton d'injection (3) par l'intermédiaire d'un élément élastique ou élément à ressort (2), **caractérisé en ce que** la tige de piston (1), l'élément élastique ou élément à ressort (2) et le bouton d'injection (3) sont d'un seul tenant.

2. Élément d'insertion selon la revendication précédente, dans lequel une zone destinée à la rupture (1a, 3a) est prévue entre la tige de piston (1) et le bouton d'injection (3).

3. Élément d'insertion selon l'une quelconque des revendications précédentes, dans lequel le bouton d'injection (3) peut coulisser ou être déplacé par rapport à la tige de piston (1).

4. Élément d'insertion selon l'une quelconque des revendications précédentes, dans lequel la tige de piston (1) comporte au moins deux éléments d'absorption de force (1a, 1b).

5. Élément d'insertion selon l'une quelconque des revendications précédentes, dans lequel le bouton d'injection (3) comporte un élément de transmission de force (3a), par lequel une force appliquée sur le bouton d'injection (3) peut être transmise à la tige de piston (1) ou à un élément (1a) de la tige de piston.

6. Dispositif d'injection comportant un boîtier (4) et un élément d'insertion (1, 2, 3) selon l'une des revendications précédentes.

7. Dispositif d'injection selon la revendication précédente, comportant au moins un élément de retenue (4a), par lequel la tige de piston (1) peut être retenue dans une position axiale à l'encontre d'une force agissant dans la direction proximale.

8. Dispositif d'injection selon l'une des deux revendications précédentes, comportant au moins un élément de refoulement (4b) qui peut repousser ou déplacer un élément ou un bras (3a) du bouton d'injection dans la direction radiale du dispositif d'injection (3), lorsqu'une force axiale est appliquée sur le bras (3a).

9. Dispositif d'injection selon l'une des trois revendications précédentes, lequel est réalisé de telle sorte que l'élément d'insertion (1, 2, 3) selon l'une des revendications 1 à 5 peut être inséré par le côté proximal dans le boîtier (4) du dispositif d'injection.
